# EUROPEAN PATENT SPECIFICATION

(11) **EP 1 136 061 B1**
(45) Date of publication and mention of the grant of the patent: **03.01.2007**
(21) Application number: 01105678.5
(22) Date of filing: 07.03.2001
(51) Int. Cl.: A61K 8/34, C11B 9/00, C07C 43/196, A61L 9/01, C11D 3/50, C11D 9/44, A61Q 13/00

(54) **Optically active, oxygenated, alicyclic compounds and their use as perfuming ingredients**
Optisch aktive, sauerstoffhaltige, alizyklische Verbindungen und ihre Verwendung als Duftstoffe
Composés oxygénés alicycliques ayant une activité optique et leur utilisation à titre d'ingrédients parfumants

(30) Priority: 20.03.2000 CH 5232000
(43) Date of publication of application: 26.09.2001
(73) Proprietor: FIRMENICH SA, 1211 Genève 8 (CH)
(72) Inventor: Margot, Christian, 1182 Gilly (CH)
(74) Representative: Salvaterra-Garcia, Maria de Lurdes

(56) References cited:
- US-A- 4 608 445
- US-A- 5 087 612

## Description

The present invention relates to the field of perfumery. More particularly, it concerns a number of optically active isomers of the compounds as claimed in claims 1-3.

The olfactory properties of compounds such as 1-(2',2',6'-trimethyl-1'-cyclohexyloxy)-2-pentanol, are described by W. Giersch et al. in US 4,608,445.

However, the prior art only mentions the named compounds in the form of diastereomeric or racemic mixtures.

Now, the compounds as claimed have a plurality of chiral centres. None of the documents of the prior art suggests any possible interest from an organoleptic point of view for a particular optically active isomer of the compounds referred to therein.

The synthesis of any pure, optically active isomer is in general difficult and costly. It is also impossible to determine a priori, even in the presence of a racemate of good olfactory quality, whether a particular pure, optically active component will have different or in some way better olfactory qualities than the racemate.

We have now discovered, quite surprisingly, that the compounds as claimed in the form of new, optically active isomers have characteristic smells which are distinct from those of the other isomers and the intensity of which can vary considerably. Furthermore, for use in perfumery, some of these optically active species have distinct advantages over known racemic mixtures.

The present invention therefore relates to a compound of the formula

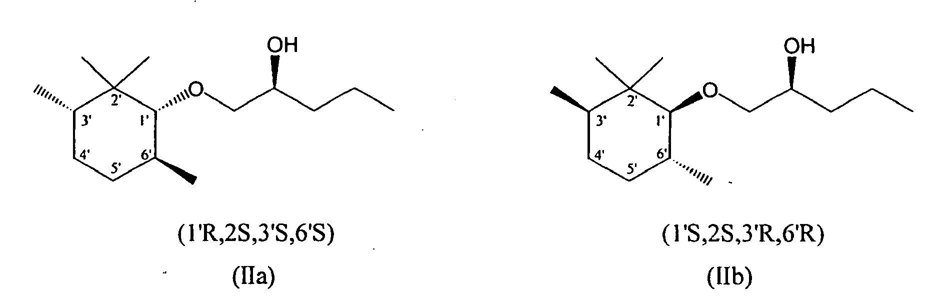

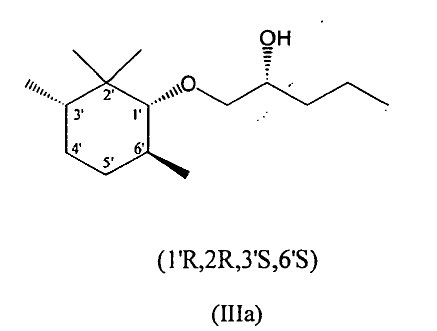

The isomer of the formula (IIa), a preferred compound according to the invention, has proved to be the best odorant ingredient of the series, possessing a fragrance which is much more intense and long-lasting than that of the other optically active isomers. Its extremely intense woody fragrance also has an amber-scented, dry, very natural character. The isomer of the formula (IIIa), which is also a highly rated compound of the invention, has similar olfactory characteristics, but its smell is less intense than that of the isomer (IIa). Generally speaking, the compounds of the invention in which the hydroxyl group has an S configuration have a more intense smell than the others. Furthermore, it has been ascertained, unexpectedly and highly advantageously, that the smell of the compound of the formula (IIa) is entirely distinguished from that of the corresponding racemate by the fact that it does not have an animal/perspiration note.

In addition, the invention also relates to optically active mixtures of diastereoisomers. From among these, the mixture of (1'R,2S,3'S,6'S)-1-(2',2',3',6'-tetramethyl-1'-cyclohexyloxy)-2-pentanol and (1'S,2S,3'R,6'R)-1-(2',2',3',6'-tetramethyl-1'-cyclohexyloxy)-2-pentanol is extremely highly regarded and represents a preferred embodiment of the invention. This mixture is appreciated for its woody, amber-scented fragrance devoid of all animal/perspiration notes. Moreover, the said mixture has organoleptic characteristics which are very different from those of the corresponding racemate, i.e. 1-(2,2,3,6-tetramethyl-1-cyclohexyloxy)-2-pentanol. Indeed, the woody, amber-scented fragrance of the mixture also has a much more amber-scented/fruity, almost floral character which is much less cedar/animal than the fragrance of the racemate. The intensity of its fragrance is also an advantage.

Another object of the invention is a method to confer, improve, enhance or modify the odour properties of a perfuming composition or a perfumed product, which method comprises the step of adding as a perfuming ingredient to said composition or product, a compound of formula (I) in the form of an optically active isomer or a mixture of these isomers.

In fact, the compounds of the invention lend themselves to use in practically all areas of the modem perfume industry, for example applications in fine perfumery, i.e. in the preparation of perfumes and eaux de toilette in which original olfactory effects can be achieved.

The compounds can also be used in functional perfumery. Examples of this type of application include soaps, shower or bath gels, shampoos or other hair-care products. cosmetic preparations, deodorants and air fresheners, liquid or solid detergents and fabric softeners for the treatment of textiles, detergent compositions or cleaning products for washing up or for cleaning a variety of surfaces.

In these applications, the compounds according to the invention can be used alone or mixed with other perfuming ingredients, solvents or additives commonly used in perfumery. The nature and variety of these co-ingredients does not call for a more detailed description here, which in any case could not be exhaustive, the skilled person being able to select them on the basis of his general knowledge and according to the nature of the product to be perfumed and the desired olfactory effect. These perfuming co-ingredients can belong to chemical classes as varied as alcohols, aldehydes, ketones, esters, ethers, acetates, nitriles, terpene hydrocarbons, nitrogenous or sulphurous heterocyclic compounds and essential oils of natural or synthetic origin. Many of these ingredients are also listed in reference texts such as the book by S. Arctander, Perfume and Flavor Chemicals, 1969, Montclair, New Jersey, USA, or its more recent versions, and in other works of a similar nature.

Their particular selection will be made so as to obtain the desired fragrance and a perfuming effect of which the duration will also depend on the function of the consumer product to be perfumed. For example, it is desirable for a detergent or fabric softener in particular not only to be able to remove stains and dirt from the treated textiles or to make them soft to the touch, but also to impart a pleasant and long-lasting fragrance to these texiles during washing because the user of these products often finds it desirable for the fragrance of the clothes or laundry to be perceptible even several days after they have been washed, dried and ironed. It has long been known to select the perfuming ingredients which have the required properties of tenacity and substantivity to produce this long-lasting perfuming effect, and the expert perfumer is capable of incorporating into the perfuming composition that he wishes to use to obtain a particular fragrance a certain number of these compounds of outstanding tenacity and substantivity and of which the smell is also compatible with the overall fragrance that he wishes to obtain.

Furthermore, it has long been part of his general knowledge that the substantivity and tenacity of the perfuming ingredients on laundry or other surfaces to be perfumed is a function of a plurality of parameters, in particular the molecular weight and the log P of the said ingredients, and also the quality of the textiles to be treated with the detergents or fabric softeners perfumed with these ingredients [see for example S. Escher et al., J. Amer. Org. Chem. Soc. 71, 31 (1994)].

The art of perfumery calls upon all this knowledge and the application thereof in the selection not only of the perfuming ingredients, the combination of which enables the desired olfactory effect to be created, but also of heavy and less volatile ingredients, the fragrance of which is perceptible for a much longer period of time than that of the lighter and more volatile compounds. Moreover, the perfumer creates the perfumes which are adapted to the characteristics of the consumer product base that he wishes to perfume and in particular are capable of covering the smell of this base and imparting to it a pleasant smell, and he uses the ingredients which are necessary and suitable for this purpose on the basis of their stability, hedonic effect, volatility and tenacity.

The proportions in which the compounds according to the invention can be incorporated into the various aforementioned products vary over a wide range of values. These values are dependent on the nature of the article or product that one wishes to perfume and on the desired olfactory effect, and also on the nature of the co-ingredients in a given composition when the compounds of the invention are mixed with perfuming co-ingredients, solvents or additives commonly used in the art.

As an example, typical concentrations are in the order of 1% to 10% by weight, even 20%, of the compounds according to the invention, based on the weight of perfuming composition into which they are incorporated. Lower concentrations can be used when the compounds are applied directly in the perfuming of the various consumer products mentioned hereinabove.

The compounds of the invention are in general prepared from a substituted cyclohexanol in the form of an optically active isomer. Therefore, the isomers of the formula (Ia) or (Ib) with the absolute configuration shown hereinabove are obtained by the condensation reaction of a suitable substituted cyclohexanol with an epoxide, these two compounds being in the form of suitable, optically active isomers, that is to say the stereochemistry of which is selected as a function of the stereochemistry of the desired final product, as illustrated in the following scheme :

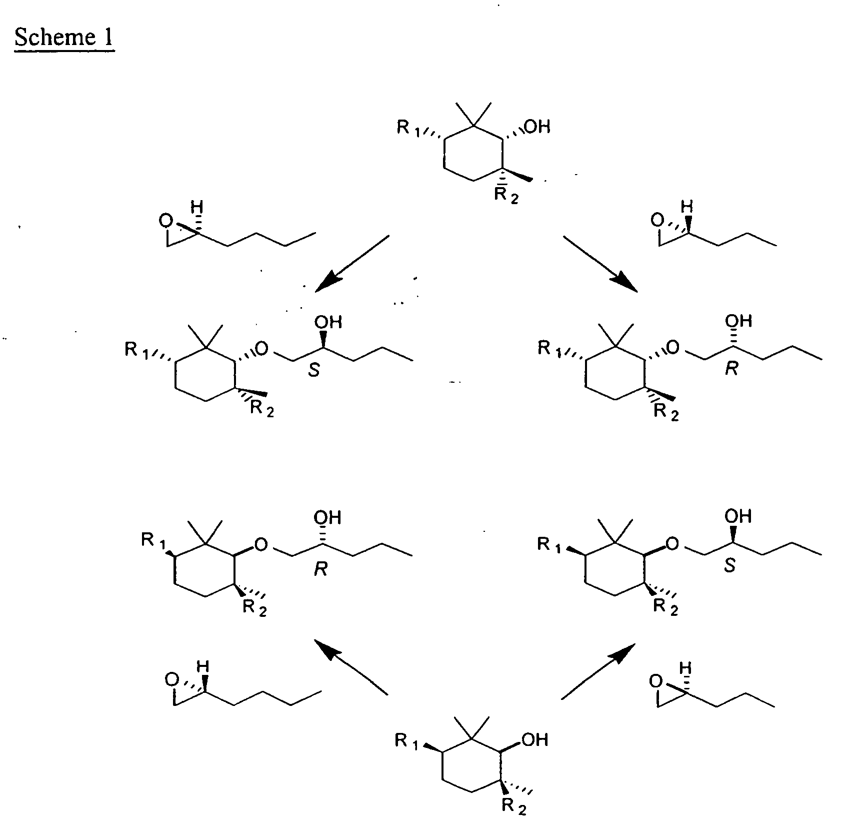

The optically active mixtures of compounds of the formulae (IVa) and (IVb) are also prepared from an optically active substituted cyclohexanol. Following an allylation reaction, then epoxidation and opening by nucleophilic oxirane with the aid of alkyl magnesium bromide, this produces an alkoxy alcohol. Oxidation of the latter produces an alkoxy ketone which, by stereoselective reduction, gives the desired mixture. The Corey reduction reaction by diborane in the presence of (S)-α,α-diphenyloxazaborolidine enables the absolute configuration of the hydroxyl function to be controlled.

The following scheme illustrates the preparation of the optically active mixtures according to the invention : 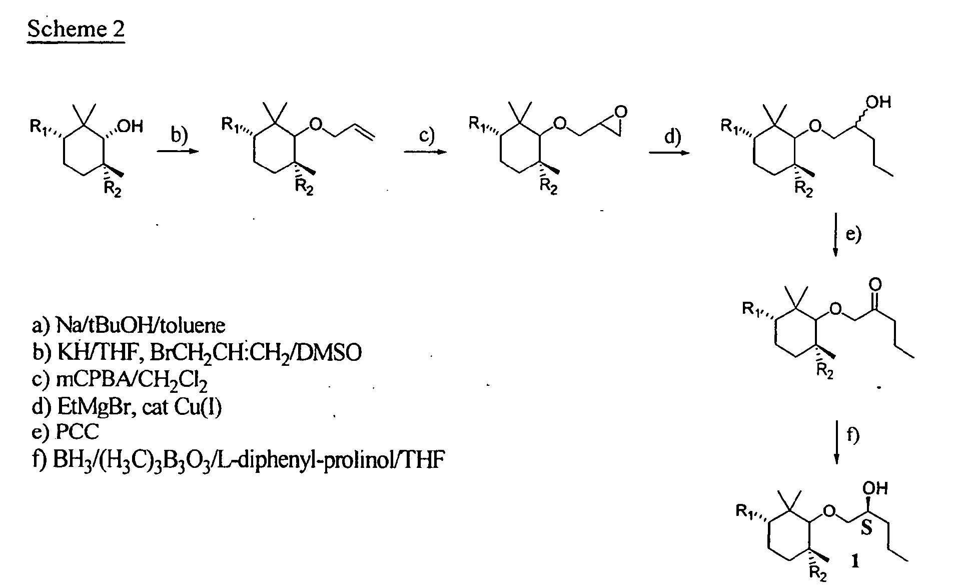
THF : tetrahydrofuran
DMSO : dimethyl sulphoxide
mCPBA : *m*-chloroperbenzoic acid
EtMgBr : ethyl magnesium bromide
PCC : pyridine chlorochromate
(H₃C)₃B₃O₃: trimethylboroxine

The preparation of the compounds according to the invention will be described in further detail in the following examples, in which temperatures are given in degrees Celsius and abbreviations have the usual meaning in the art. The invention will also be illustrated by examples describing the use of the compounds of the invention in perfumery.

### Example 1

### Stereoselective preparation of the four optically active isomers of 1-(2,2,6-trimethyl-1-cyclohexyloxy)-2-pentanol

### General method for obtaining the starting materials

Dihydrocyclocitral was obtained as a *ca* 90:10 mixture of *trans* and *cis* isomers upon acidic treatment of citronellal enol acetate according to the method described by D.P. Simmons et al. in Helv. Chim. Acta (1988), 71, 1000, the content of which is hereby included by reference. When (-)-S-citronellal was used as starting material for this cyclization, (+)-(1R, 6S)-2,2,6-trimethylcyclohexane-1-carbaldehyde and (-)-(1S, 6S) *cis* isomer were obtained in 80-85% yield. The corresponding enantiomers were obtained from (+)-R-citronellal.

### 1. (+)-(1R,6S)-2,2,6-Trimethylcyclohexanol and (-)-(1S,6R)-trimethylcyclohexanol

### a) (+)-(1R,6S)-2,2,6-Trimethylcyclohexyl formate

A solution of 9.73 g (63.0 mmol) of (+)-(1R, 6S)-2,2,6-trimethylcyclohexane-1-carbaldehyde was reacted with 23.5 g of 70% pure 3-chloro-perbenzoic acid (95 mmol) in 100 ml of dichloromethane at 25° during 4 days. Upon complete conversion as monitored by GLC (gas-liquid chromatography) (Supelcowax^{®} column, 15 m, 70°C, 10°/min up to 220°), the mixture was poured onto 500 ml of water. Decantation, extraction with 2x50 ml of dichloromethane, rinsing with 2x100 ml of brine, drying over sodium sulfate, evaporation at ambient pressure and Kugelrohr distillation afforded 9.0 g of 97% pure material. Integration of the ¹H-NMR signals showed that *ca* 6-8% *cis* isomer was present. Yield 82%.
Analytical data :
- [α_{D}²⁰] =: + 37.4 (CHCl₃, 4%)
- IR: 2927(*s*); 1719(*s*); 1459(*m*); 1388(*w*); 1366(*m*) ; 1186, 1167(*s*); 945(*s*).
- MS: 170 (1%, M⁺); 124(46%); 109(100%); 95(25%); 82(98%); 69(39%).
- ¹H-NMR: 8.20(s, 1H); 4.50(*d*, *J*=11, 1H); 1.7(*m*, 2H); 1.45(*m*, 3H); 1.3(*m*, 1H); 1.05(*m*, 1 H) ; 0.95(*s*, 3H); 0.88(*s*, 3H); 0.84(*d*, *J*=6, 3H).
- ¹³C-NMR: 161.3 (*d*); 84.5(*d*) ; 39.5(*t*); 35.3(*s*); 34.1(*t*); 32.6(*d*); 29.1(*q*); 21.2(*t*); 19.3(*q*); 18.7(*q*).

### b) (-)-(1S,6R)-2,2,6-Trimethylcyclohexyl formate

The reaction of (-)-(1S, 6R)-2,2,6-trimethylcyclohexane-1-carbaldehyde with 3-chloro-perbenzoic acid was run in the same conditions as described under *a)* to yield 85% of (-)-(1S,6R)-2,2,6-trimethylcyclohexyl formate.
Analytical data:
[α_{D}²⁰] = - 37.0 (CHCl₃, 4%)
Spectra identical to those of the enantiomer.

### c) (+)-(1R,6S)-2,2,6-Trimethylcyclohexanol

A solution of 6.05 g (35.6 mmol) of (6S)-2,2,6-trimethylcyclohexyl formate in 50 ml methanol was heated under reflux with 6.0 g (106 mmol) of potassium hydroxyde in 18 ml of water during 1 h. GC (SP-2100, 100°C, 15°C/min to 220°C) showed complete conversion. The reaction mixture was cooled to room temperature, partitioned with 100 ml of diethyl ether and the aqueous phase was extracted twice with 50 ml of diethyl ether. The dried organic phases were evaporated, the concentrate filtrated over 50 g silica (CH₂Cl₂) to yield 4.90 g of pure material. Yield 95%, chemical purity >99% (GC), trans:cis ratio *ca* 95:5 (¹H-NMR), enantiomeric purity 97% (GC: 25 m Megadex, 100°C, 0.5°C/min to 130°C).
Analytical data :
- [α_{D}²⁰] =: + 28.1 (CHCl₃, 4%)
- IR: 3380 (*s*, broad) ; 2945, 2921, 2864, 2845(*s*); 1454(*s*); 1364(*s*); 1040(*s*); 952(s).
- MS: 142 (87%, M⁺); 124(22%) ; 109(100%); 95(58%) ; 82(96%); 81(55%); 71(66%).
- ¹H-NMR: 2.82(*d*, *J*=8.5, 1H); 1.69(*ddq*, *J*=3+3+13, 1H); 1.4-1.5(*m*, 5H); 1.2(*m*, 2H) ; 0.98(*s*, 3H) ; 0.97(*d*, *J*=8, 3H) ; 0.88(*s*, 3H).
- ¹³C-NMR: 83.6(*d*); 39.9(*t*); 35.7(*s*); 34.7(*d*); 34.6(*t*); 29.5(*q*); 21.5(*t*); 19.2(*q*); 18.3(*q*)

### d) (-)-(1S,6R)-2,2,6-Trimethylcyclohexanol

In analogy to the above c) description, (-)-(1S, 6R)-2,2,6-trimethylcyclohexanol was obtained in 92% yield and 99% chemical purity; trans:cis ratio *ca* 95:5 (¹H-NMR), enantiomeric purity 98% ee (GC: 25 m Megadex, 100°C, 0.5°C/min to 130°C).
Analytical data:
[α_{D}²⁰] = + 28.1 (CHCl₃, 4%)
Spectra identical to those of the enantiomer.

### 2. 1-(2,2,6-Trimethyl-1-cyclohexyloxy)-2-pentanol isomers

### General coupling procedure :

Under argon atmosphere, 2.7 g (23 mmol) of potassium hydride dispersion 35% in mineral oil was washed twice with 20 ml of anhydrous pentane and decanted before it was suspended in 30 ml of dry tetrahydrofuran (THF). To this suspension, 2.20 g (15.5 mmol) of (+)-(1R, 6S)-2,2,6-trimethyl-cyclohexanol in 5 ml of THF were added dropwise over 10 min. The reaction was stirred 2 hours before adding 2.0 ml of DMPU. The mixture was heated to reflux (bath 80°C) and 1.73 g (20.1 mmol) of (S)-1,2-epoxypentane were added in one portion. The reaction was kept at 80°C overnight until approx. 90% of the starting trimethyl-cyclohexanol were converted according to GLC (15 m SP-2100, 100°C, 15°C/min to 220°C). The reaction mixture was poured onto 100 ml of ice water, partitioned and extracted twice with 50 ml of pentane. The combined organic phases were washed twice with 100 ml of brine, dried over sodium sulfate and the solvent evaporated. The crude oil, 4.2 g, was eluted with 9:1 cyclohexane /ethyl acetate on 250 g silica. A bulb-to-bulb distillation of the concentrate of the pure fractions (110°C oven, 0.1 mmHg) afforded 0.85 g of (+)-(1'R, 2S, 6'S)-1-(2',2',6'-trimethyl-1'-cyclohexyloxy)-2-pentanol as a colourless liquid. Yield 23% based on 2,2,6-trimethyl-cyclohexanol. This protocol has also been applied to following reactions: (+)-trimethyl-cyclohexanol with (R)-epoxypentane, (-)-trimethyl-cyclohexanol with (S)-epoxypentane, respectively (R)-epoxypentane.

*The following two enantiomers have identical spectra:*
**(+) (1'R, 2S, 6'S)-1-(2',2',6'-trimethyl-1'-cyclohexyloxy)-2-pentanol**
   [α_{D}²⁰] = + 30.0 (CHCl₃, 4%)
**(-) (1'S, 2R, 6'R)-1-(2',2',6'-trimethyl-1'-cyclohexyloxy)-2-pentanol**
   [α_{D}²⁰] = - 29.7 (CHCl₃, 4%)

- IR: 3444 (*w*, broad) ; 2922, 2865(*s*) ; 1454(*s*) ; 1380, 1364(*m*) ; 1093(*s*)
- MS: 228 (100%, M⁺) ; 213(3%); 157(38%); 142(20%); 125(47%); 109(32%); 82(60%); 69(67%)
- ¹H-NMR: 3.8(*m*, 1H); 3.59(*dd*, *J*=3+9, 1H); 3.4(*m*, 1H); 2.48(*d*, *J*=10,1H); 2.45(*d*, *J*=4, 1H) ; 1.3-1.7(*m*, 10H); 1.2(*m*, 1H); 0.99(*s*, 3H); 0.96(*d*, *J*=6, 3H); 0.93(t, *J*=7, 3H) ; 0.89(s, 3H)
- ¹³C-NMR: 92.3(*d*) ; 78.3(*t*) ; 70.7(*d*); 40.2(*t*) ; 36.9(*s*); 35.2(*t*) ; 34.8(*d&t*); 30.1(*q*); 21.5(*t*); 19.5(*q*) ; 19.4(*q*) ; 18.8(*t*); 14.1(*q*)

*The following two enantiomers have identical spectra:*
**(+) (1'R, 2R, 6'S)-1-(2',2',6'-trimethyl-1'-cyclohexyloxy)-2-pentanol**
   [α_{D}²⁰] = + 26.3 (CHCl₃, 4%)
**(-) (1'S, 2S, 6'R)-1-(2',2',6'-trimethyl-1'-cyclohexyloxy)-2-pentanol**
   [α_{D}²⁰] = -23.9 (CHCl₃, 4%)

- IR: 3426 (*w*, broad) ; 2923, 2865(*s*); 1454(*s*) ; 1380, 1364(*m*) ; 1093(*s*)
- MS: 228 (100%, M⁺); 213(3%); 157(33%); 142(19%); 125(44%); 109(31%); 95(16%); 87(20%) ; 82(53%) ; 69(56%)
- ¹H-NMR: 3.8 (*m*, 1H) ; 3.5(*m*, 2H) ; 2.54(*d*, *J*=3, 1H); 2.47(*d*, *J*=10, 1H) ; 1.3-1.7(*m*, 10H); 1.2(m, 1H); 0.97(*d*, *J*=6, 3H) ; 0.96(s, 3H); 0.93(*t*, *J*=7, 3H) ; 0.88(*s*, 3H)
- ¹³C-NMR: 92.4(*d*); 78.3(*t*); 70.7(*d*); 40.1(*t*); 36.8(*s*); 35.3(*t*); 34.9(*d&t*); 30.1(*q*); 21.5(*t*); 19.5(q); 19.3(*q*); 18.8(*t*); 14.2(*q*)

### Example 2

### Stereoselective preparation of the four optically active isomers of 1-(2,2,3,6-tetramethyl-1-cyclohexyloxy)-2-pentanol

### 1. (+)-(1R,3S,6S)-2,2,3,6-Tetramethylcyclohexanol

### a) (1S,2R,5S)-2-Isopropenyl-5-methylcyclohexanol [(+)-isopulegol]

A 2 1 flask fitted with a mechanical stirrer and in an argon atmosphere was filled with 250 g of (-)-S-citronellal (enantiomeric excess > 98%) (1.62 mol) and 0.8 1 of anhydrous toluene. The mixture was cooled to -5° in a bath of acetone and ice while 365 g of anhydrous zinc bromide (1.63 mol) were added in 10 portions over 2.5 h. The mixture was stirred for a further 2 h at -5° until conversion was complete, as indicated by GLC (gas-liquid chromatography) (Carbowax column, 15 m, 100°, 15°/min. up to 220°). The reaction mixture was then filtered. The filtrate was washed with brine, then dried over sodium sulphate. Evaporation under a vacuum of 2.6 x 10³ Pa produced 262 g of raw concentrate. A sample of the raw oil was purified by filtration over silica (cyclohexane / ethyl acetate, 9:1).
Analytical data:
- IR (GC-FTIR): 3390(*m*, broad); 3071(*w*); 1644(*s*); 1447(*s*); 1374(*s*); 1094(*s*) ; 1050(*s*) ; 1025(*s*).
- MS: 154(16%, M⁺); 139(20%) ; 136(40%); 121(65%); 111(34%); 95(60%); 41(100%).
- ¹H-NMR (CDCl₃, 360MHz): 4.88(*t*, J=2, 1H); 4.85(*s*, 1H); 3.46(*dt*, J=4+10, 1H); 2.06(*s*, 1H); 2.03(*d*, J=14, 1H); 1.88(*ddd*, J=3+10+14. 1H); 1.71(*s*, 3H) ; 1.7(*m*, 2H) ; 1.5(*m*, 1H); 1.32(*dq*, J=4+14, 1H); 1.0(*m*, 2H); 0.94(*d*, J=8, 3H).
- ¹³C-NMR (CDCl₃, 90.6MHz): 146.7(*s*); 112.8(*t*); 70.4(*d*); 54.1(*d*); 42.8(*t*); 34.4(*t*); 31.5(*d*); 29.7(*t*) ; 22.2(*q*); 19.2(*q*).

### b) Mixture of (-)isopulegone and (-)pulegone

A 3 1 flask in an argon atmosphere and fitted with a mechanical stirrer was filled with 440 g of pyridine chlorochromate (2.0 mol), 440 g of celite and 1.4 1 of methylene chloride. 260 g of raw isopulegol obtained under *a)* were added dropwise over a period of 3 h to the mixture held at a temperature of 15° by means of a water bath. After this addition, the mixture was stirred at ambient temperature for 5 h and conversion was monitored by gas-liquid chromatography (SP-2100 column, 15 m, 100°, 15°/min. up to 220°). The suspension obtained was filtered, and the filtrate eluted over 300 g of silica with dichloromethane. Concentation under vacuum (2 x 10³ Pa, bath temperature approximately 40°) produced 207 g of a raw oil which was fractionated under vacuum (Vigreux column, 20 cm). The fraction distilling at 49° under 13.3 Pa weighed 130 g and contained only 2 products in a ratio of 57:43, identified as isopulegone and pulegone respectively by comparison of their spectra with the authentic product.
The yield was 53% based on the (S)-citronellal.
Analytical data:
- IR (GC-FTIR): *1^{st} peak :* 3080(*w*) ; 2962, 2939, 2885(*s*) ; 1729(*s*) ; 1646(*w*); 1454(*m*).
*2^{nd} peak*: 2964, 2930(*s*); 1699(*s*); 1622(*m*);1448(*w*); 1382(*w*); 1282(*w*).
- MS: *1^{st} peak :* 152(46%, M⁺); 137(23%); 123(95%); 109(100%); 95(30%) ; 93(95%); 81(43%); 67(97%).
*^{2nd} peak :* 152(69%, M⁺); 137(28%); 109(41%); 95(18%); 81(100%); 67(81%).

### c) (3S, 6R)-6-Isopropenyl-2,2,3,6-tetramethylcyclohexanone and (3S, 6S)-6-isopropenyl-2,2,3,6-tetramethylcyclohexanone

A 3 1 flask in an argon atmosphere and fitted with a mechanical stirrer was filled with I 1 of anhydrous tetrahydrofuran (THF) and 150 g of potassium hydride paste at 35% in mineral oil (approximately 1.3 mol). The suspension was cooled to between 15° and 20°. The temperature was held at this level while 50 g of the mixture obtained under *b*) were added dropwise over a period of 1.5 h. The mixture was then stirred for 4 h and allowed to heat up to ambient temperature. The mixture was then re-cooled to a temperature between 15° and 20° and 71 ml of methyl iodide (1.15 mmol) were added dropwise over a period of 1 h. The reaction mixture was allowed to heat up to ambient temperature, then the mixture was stirred overnight. The reaction medium was then carefully poured over 400 ml of iced water. After decanting, the aqueous phase was extracted with 2 x 100 ml of dichloromethane. The organic phases were washed with 300 ml of brine and dried over anhydrous sodium sulphate. The solvents were evaporated (30° / 1.3 x 10³ Pa) and the oily residue distilled under vacuum (58°-62°, 40 Pa). A fraction of 32 g containing 91% of a mixture of diastereomers (80:20) of (3S)-6-isopropenyl-2,2,3,6-tetramethylcyclo-hexanone was obtained. Yield 46%.
Analytical data :
- IR: 3080(*w*) ; 2965, 2929, 2872(*s*); 1696(*s*); 1634(*m*) ; 1456(*s*); 1370(*s*).
- MS: *Peak 1* : 194(8%, M⁺); 179(3%); 137(23%); 123(80%); 107(31%); 96(3 1 %) ; 82(100%).
*Peak 2* : 194(8%, M⁺); 179(3%); 137(19%); 123(68%); 107(32%); 96(33%); 82(100%).
- ¹H-NMR (CDCl₃, 360MHz): *Major isomer :* 4.86(*s*, 1H); 2.15(*m*, 1H); 2.0(*m*, 2H); 1.70(*s*, 3H); 1.65(*m*, 2H); 1.45(*m*, 1H); 1.14(*s*, 3H); 1.13(*s*, 3H) ; 0.98(*s*, 3H) ; 0.88(*d*, J=6.7, 3H).
*Minor isomer:* 4.97(*s*, 1H); 4.91(*s*, 1H) ; 2.3(*m*, 1H) ; 1.64(*s*, 3H) ; 1.11(*s*, 3H) ; 1.08(*s*, 3H); 0.95(*s*, 3H) ; 0.93(*d*, J=6, 3H).
- ¹³C-NMR (CDCl₃, 90.6MHz): *Major isomer:* 218.7(*s*) ; 147.6(*s*); 110.5(*t*); 53.3(*s*) ; 48.4(*s*) ; 37.8(*d*);31.1(*t*); 26.1(*q*) ; 26.0(*q*) ; 25.8(*t*); 21.9(*q*) ; 20.9(*q*) ; 16.1(*q*).
*Minor isomer :* 216.8 ; 147.0 ; 110.4 ; 53.5 ; 50.3 ; 42.9 ; 36.0 ; 27.1 ; 27.0 ; 24.0 ; 19.6; 16.2.

### d) (3S,6R)-6-Acetyl-2,2,3,6-tetramethylcyclohexanone and (3S,6S)-6-acetyl-2,2,3,6-tetramethylcyclohexanone

A 500 ml Schlenk tube was filled with 58 g of a mixture of diastereomers (3S,6R)-6-isopropenyl-2,2,3,6-tetramethylcyclohexanone and (3S,6S)-6-isopropenyl-2,2,3,6-tetramethylcyclohexanone (0.30 mol) obtained under c) in 240 ml of dichloromethane. The tube was cooled to a temperature of -78° and a stream of ozone (2.5 g/h) in 50 l/h of oxygen was bubbled into the solution by means of sintered glass for almost 6 h until the persistence of a blue colour (approximately 0.3 mol). After the tube has been purged and degassed using nitrogen, the reaction mixture was allowed to heat up to ambient temperature. The mixture was then poured over 350 ml of a solution of sodium bisulphite at 10% (350 g, 0.33 mol) and stirred overnight. The phases were separated and the aqueous phase was extracted twice with 100 ml of dichloromethane. The combined organic phases were washed with 100 ml of brine, then dried over anhydrous sodium sulphate. The solution was used such as it was for the following step.

### e) (+)-(3S)-2,2,3,6-Tetramethylcyclohexanone

A 2 1 flask was filled with 700 ml of methanol and 70 g of potassium hydroxide pellets (1.25 mol) introduced in one go and dissolved by mechanical stirring at ambient temperature. The product of ozonolysis in dichloromethane, obtained above, was added dropwise to the mixture over a period of 1 h. After this addition, the dichloromethane was distilled by heating using a water bath. The resulting mixture was heated to reflux over a period of 2 h (bath temperature 90°), then the majority of the methanol was distilled. The cold residue was poured over 300 ml of iced water and decanted. The aqueous phase was extracted with 2 x 50 ml of pentane, then the combined organic phases were dried over anhydrous sodium sulphate and the solvents were distilled. Brief distillation of the residue finally produced 33 g of a colourless oil, with a yield of 67% over the two steps (based on (3S,6R)-6-isopropenyl-2,2,3,6-tetramethylcyclohexanone and (3S,6S)-6-isopropenyl-2,2,3,6-tetramethylcyclohexanol). The incorporation of the signal ¹H-NMR showed that the product was an 85:15 mixture of two diastereoisomers, namely the ketones *trans-*(+)-(3S,6S)-2,2,3,6-tetramethylcyclohexanone and *cis-*(3S,6R)-2,2,3,6-tetramethylcyclohexanone.
Analytical data:
- [α_{D}²⁰] = +65.5: (c=0.045; CHCl₃)
- IR: 2968, 2928, 2859(s); 1702(s); 1450(s); 1387, 1373(m).
- MS: (mixture of non-separated *cis*/*trans* isomers) 154 (50%, M⁺); 112(27%); 96(100%); 84(38%); 69(73%).
- ¹H-NMR (CDCl₃, 360MHz): *trans*, *major isomer : 2.65(ddq,* J=6.5+6.5+13, 1H); 2.0(*m*, 2H); 1.6(*m*, 2H); 1.29(*dq*, J=4+13, 1H); 1.05(*s*, 3H) ; 1.02(*s*, 3H) ; 0.98(*d*, J=6.5, 3H) ; 0.95(*d*, J=6.0, 3H). *cis, minor isomer : 2.71 (ddq,* J=6.5+6.5+13,1H); 2.22(*tt*, J=4.5+13, 1H); 1.5-1.7(*m*, 3H); 1.4(*m*, 1H); 1.43(*s*, 3H); 1.26(*s*, 3H); 0.99(*d*, J=6, 3H); 0.86(*d*, J=7, 3H).
- ¹³C-NMR (CDCl₃, 90.6MHz): *trans*, *major isomer :* 217.3(*s*) ; 48.5(*s*) ; 43.1(*d*) ; 40.0(*d*); 35.2(*t*) ; 30.2(*t*) ; 22.6(*q*) ; 19.0(*q*) ; 15.7(*q*) ; *15.1 (q). cis, minor isomer :* 218.0, 49 ; 42.3 ; 31.2 ; 28.1 ; 26.9 ; 22.2 ; 16.0.

### f) (+)-(1 R, 3S, 6S)-2,2,3,6-Tetramethylcyclohexanol

The reaction was carried out in a 1.5 1 double-walled flask fitted with a mechanical stirrer, an adding funnel, a condenser and a thermometer in an argon atmosphere, in which 250 ml of toluene and 15.0 g of metallic sodium (0.65 mol) were heated to reflux for a period of 15 min. The molten two-phase toluene/metal mixture was cooled during vigorous stirring (mechanical stirring with Medimex^{®} transmission) and the metal solidified in the form of small spheres. At 0°, a solution of 33 g of (+)-(3S)-2,2,3,6-tetramethylcyclohexanone (0.21 mol) in 60 g of isopropanol (1.0 mol) was added dropwise to the metallic preparation over a period of 3 h. The reaction mixture was stirred at 0° overnight, then gas-liquid chromatography (Supelcowax, 100°-200°, 15°/min.) showed a conversion of 95%. The remaining sodium was destroyed by the slow addition of ethyl alcohol, then the mixture was added to 200 ml of water. The organic and aqueous phases were separated and the aqueous phase extracted with 2 x 100 ml of toluene. The combined organic phases were washed with 200 ml of brine, dried using sodium sulphate and concentrated at 5.3 x 10³ Pa to produce 38 g of concentrate. Elution over 500 g of silica with a 95:5 cyclohexane / ethyl acetate eluent mixture, followed by double crystallisation, produced 19.5 g of pure (+)-(1R,3S,6S)-2,2,3,6-tetramethylcyclohexanol with a yield of 70% and an enantiomeric excess > 98% according to gas-liquid chromatography (Megadex 5 column, 15 m, 70°, 2°/min. up to 200°).
Analytical data:
- [α_{D}²⁰] = +16.9: (c=0.039; CHCl₃
- IR: 3384 *(m, broad*); 2955, 2912, 2860(*s*); 1454(*s*); 1095(*s*); 1011(*s*).
- MS: 156 (84%, M⁺); 138(20%); 123(100%); 113(37%); 109(36%); 95(55%).
- ¹H-NMR (CDCl₃, 360MHz): *2.77(dd,* J=5.5+10.5, 1H); 1.65(*dq*, J=3.5+12.5, 1H) ; 1.54(*broad d,* J=5, 1H, disappears with D₂O); *1.5(m,* 1H); 1.1-1.4(*m*, 3H); 1.0(*m*, 1H); 0.99(*s*, 3H); 0.97(*d*, J=7.5, 3H); *0.85(d,* J=6.5, 3H) ; 0.71(s, 3H).
- ¹³C-NMR (CDCl₃, 90.6MHz): 84.0(*d*); 40.8(*d*) ; 39.0(*s*); 34.3(*d*); 33.5(*t*); 30.2(*t*); 25.8(*q*) ; 19.4(*q*) ; 15.8(*q*) ; 12.5(*q*).

### 2. (-)-(1S,3R,6R)-2,2,3,6-Tetramethylcyclohexanol

(-)-(1S,3R,6R)-2,2,3,6-Tetramethylcyclohexanol was prepared in six steps starting from R-(+)-citronellal (enantiomeric excess > 98%) in the same way as described under **1**. The compounds (-)-isopulegol, (+)-isopulegol/ (+)-pulegone, (3R)-6-isopropenyl-2,2,3,6-tetramethylcyclohexanone, (3R)-6-acetyl-2,2,3,6-tetramethyl-cyclohexanone, (-)-3R-2,2,3,6-tetramethylcyclohexanone and (-)-(1S,3R,6R)-2,2,3,6-tetramethylcyclohexanol were thus successively obtained. All their spectral data are identical to that of their respective enantiomers. (-)-3R-2,2,3,6-Tetramethylcyclohexanone had an α_{D}²⁰ = -66.2 (c=0.045; CHCl₃). (-)-(1S,3R,6R)-2,2,3,6-tetramethylcyclohexanol had an ee > 98% according to gas-liquid chromatography for resolution of the enantiomer (Megadex 5 column, 15 m, 70°, 2°/min. up to 200°) and α_{D}²⁰ = -17.4 (c=0.039; CHCl₃).

### 3. 1,2-Epoxypentanes

### a) (S)-2-Chloro-1-pentanol

48.5 g of L-norvaline [α²⁰_{D} = +30 (c=10; HCl aq. at 20%)] (0.41 mol) were dissolved in 700 ml of HCl 6N and the resulting solution was cooled to -10°. During vigorous stirring, 46.4 g of sodium nitrite (0.67 mol) were added over a period of 10 min. while the temperature was held between -5° and -10°. After 15 hours' stirring at -5°, the reaction was extracted with 3 x 100 ml of pentane. The combined organic phases were washed with 200 ml of brine, dried over anhydrous sodium sulphate and concentrated under reduced pressure, finally producing 48 g of raw oil.

This oil was dissolved in 50 ml of anhydrous diethyl ether and the suspension was added dropwise to a suspension of 12.5 g of lithium aluminium hydride (330 mmol) in 200 ml of diethyl ether at 10°. The mixture was carefully poured over 250 ml of iced sulphuric acid at 10%. After the organic phase was decanted, the aqueous phase was saturated with sodium chloride and extracted twice with 50 ml of diethyl ether. The combined organic extracts were dried over anhydrous sodium sulphate and concentrated at atmospheric pressure. Fractionation in a 15 cm Vigreux column produced 27.3 g of colourless liquid with a boiling point between 88° and 90° for 2 x 10³ Pa. The yield was 53% based on the norvaline.
Analytical data:
- IR: 3320 (*s*, broad) ; 2925(*s*); 1455(*m*) ; 1379(*m*); 1275(*m*); 1200(*m*) ; 1050(*s*, broad).
- MS: 90(16%); 68(34%); 55(100%); 45(14%); 41(72%); 39(44%).
- ¹H-NMR (CDCl₃, 360MHz): 4.0(*m*, 1H); 3.78(*dd*, J=3.5+13, 1H); 3.66(*dd*, J=7+13, 1H); 1.4-1.8(*m*, 5H); 0.94(*t*, J=7. 31-1).

### b) (+)-(R)-1,2-Epoxypentane

### (i) Purification of (S)-2-chloro-1-pentanol

A solution of 26 g of (S)-chloro-1-pentanol (0.21 mol) in 33 g of pyridine (0.42 mol) and 500 ml of toluene was cooled to 0° and a solution of 57.5 g of 3,5-dinitrobenzoyl chloride (0.25 mol) was added in 3 portions over a period of 20 min. at 0°. The mixture was left overnight at ambient temperature, then poured over 300 ml of cold water. The combined organic extracts arising from partition and extraction with 2 x 50 ml of toluene were dried over sodium sulphate and concentrated under reduced pressure. The raw product was recrystallised 5 times starting from isopropyl ether / ethanol 2:1 to produce 26 g of crystalline powder with a melting point of 66°-68°; (yield of 39% based on the (S)-2-chloro-1-pentanol).

A mixture of 24 g of dinitrobenzoate (76 mmol), 250 ml of methanol and 1 ml of a solution of sodium methoxide at 30% in methanol was left for 1 h at ambient temperature. The pink solution was then poured over 250 ml of brine. After decanting and extraction with 3 x 50 ml of diethyl ether, the combined organic phases were dried over sodium sulphate and the solvent distilled at ambient pressure. Bulb-to-bulb distillation produced 8.1 g of pure (S)-2-chloro-1-pentanol.

### (ii) Closure of the chlorohydrin cycle

6.95 g of the 2-chloroalcohol (56.6 mmol) were cooled to 0°, and 6.4 g of freshly ground potassium hydroxide (0.11 mol) were added in one go. The mixture was stirred for 1 h at ambient temperature, then underwent bulb-to-bulb distillation at atmospheric pressure. Redistillation starting from calcium hydride produced 4.7 g of epoxide (furnace temperature 90°). Yield 95%.
Analytical data:
- [α]_{D}²⁵ =+16.0: (c=0.50; CHCl₃)
- IR: 2925(*s*), 1455(*m*), 1403(*m*), 1378(*m*), 1255(*m*), 1130(*s*).
- MS: 71 (70%) ; 55(15%); 43(16%); 41(100%).
- ¹H-NMR CDCl₃, 360MHz): (2.91(*m*, 1H); 2.75(*t*, J=3, 1H); 2.47(*dd*, J= 3+5, 1H) ; 1.51(*m*, 4H); 0.98(*t*, J=7, 3H).

### c) (-)-(S)-1,2-Epoxypentane

The conversion of D-norvaline [α²⁰_{D} = -30 (c=10; HCl aq. at 20%)] to (R)-2-chloropentanol and (-)-(S)-1,2-epoxypentane was carried out by a method analogous to that described under 3.
- [α]_{D}²⁵ = -15.8: (c=0.50; CHCl₃).

### 4. 1,2-Epoxybutanes

### a) (+)-(R)-1,2-Epoxybutane

The conversion of L-2-aminobutyric acid to (S)-2-chlorobutanol and (+)-1,2-epoxybutane was carried out as described hereinabove. The spectral data corresponds to that published by U. Goergens and M.P. Schneider in Tetr. Asym. 1992, 3, 1149 and by M.J. Kim and G.M. Whitesides in J. Am. Chem. Soc. 1988, 110, 2959.

### b) (-)-(S)-1,2-Epoxybutane

The conversion of D-2-aminobutyric acid to (R)-2-chlorobutanol and (-)-(S)-1,2-epoxybutane was carried out as described hereinabove. The spectral data corresponds to the values published by U. Schmidt et al in Chem. 1980, 92, 201.

### 5. Isomers of 1-(2,2,3,6-tetramethyl-1-cyclohexyloxy)-2-pentanol

### General protocol

In an argon atmosphere, 4.0 g of a dispersion of potassium hydride at 20% in mineral oil (20 mmol) were washed twice with 20 ml of anhydrous pentane and decanted before being suspended in 30 ml of anhydrous tetrahydrofuran. 2.10g of (+)-(1R,3S,6S)-2,2,3,6-tetramethylcyclohexanol in 30 ml of THF were added dropwise to this suspension over a period of 30 min. The reaction mixture was stirred for 2h before the addition of 2 ml of 1,3-dimethyl-3,4,5,6-tetrahydro-2(lH)-pyrimidone (DMPU). The mixture was heated to reflux (bath at 80°) and 1.50 g of (S)-1,2-epoxypentane (17.4 mmol) were added in one go. The reaction mixture was held at 80° until more than 90% of the starting tetramethylcyclohexanol had been converted according to gas-liquid chromatography (SP-2100 column, 15 m, 100°, 15°/min. up to 220°). The reaction mixture was then poured over 250 ml of iced water, decanted and extracted twice with 50 ml of pentane. The combined organic phases were washed twice with 100 ml of brine, dried over sodium sulphate and the solvent evaporated. Bulb-to-bulb distillation (furnace at 110°, 13.3 Pa) produced 2.5 g - 3.1 g of a colourless liquid which was eluted with a 9:1 cyclohexane / ethyl acetate mixture over 200 g of silica. Between 1.3 g and 1.5 g of pure, optically active 1-(2',2',3',6'-tetramethyl-1'-cyclohexyloxy)-2-pentanol were obtained, with a yield of 40% - 46% based on the 2,2,3,6-tetramethylcyclohexanol. For each of the isomers, the cyclohexanol and the epoxide were selected as a function of the desired final stereochemistry.
Analytical data :
**(+)-(1'R,2S,3'S,6'S)-1-(2',2',3',6'-Tetramethyl-1'-cyclohexyloxy)-2-pentanol (IIa)**
   - [α_{D}²⁰] =: + 25.5° (c=0.041 ; CHCl₃)
   - MS: 242 (60%, M⁺); 157(25%); 138(100%); 123(42%); 109(22%).
   - IR (film): 3436 *(w, broad*) ; 2955, 2914, 2866(*s*); 1452(*s*); 1098(*s*).
   - ¹H-NMR (CDCl₃, 360MHz): 3.80(*ddd*, J=3+7.5+15.5, 1H); *3.59(dd,* J=3+8, 1H); 3.40(*t*, J=9, 1H); 2.46(*d*, J=3, 1H); 2.42(*d*, J=10, 1H); 1.1-1.7(*m*, 8H) ; 1.0(*m*, 2H) ; 0.98(*s*, 3H) ; 0.95(*d*, J=7, 3H); 0.94(*t*, J=7, 3H) ; 0.82(*d*, J=6, 3H) ; 0.75(*s*, 3H).
   - ¹³C-NMR (CDCl₃, 90.6MHz): 92.7(*d*); 78.3(*t*); 70.6(*d*); 40.9(*d*); 40.2(*s*); 35.1(*d*); 34.7(*d*) ; 33.7(*t*) ; 30.2(*t*) ; 26.2(*q*) ; 15.6(*q*); 18.8(t); 15.6(*q*); 14.1(*q*) ; 13.6(*q*).
**(-)-(1'S,2R,3'R,6'R)-1-(2',2',3',6'-Tetramethyl-1'-cyclohexyloxy)-2-pentanol** (IIIb)
   - [α_{D}²⁰] =: -26.2° (c=0.042 ; CHCl₃)
   spectral data identical to (IIa)
**(+)-(1'R,2R,3'S,6'S)-1-(2',2',3',6'-Tetramethyl-1'-cyclohexyloxy)-2-pentanol** (IIIa)
   - [α_{D}²⁰] =: + 22.7° (c=0.039 ; CHCl₃)
   - MS: 242 (55%, M⁺) ; 157(25%); 138(100%); 123(42%); 109(21%).
   - IR (film): 3454 (*w*, *broad*); 2955+2913+2867(*s*); 1452(*s*); 1098(*s*).
   - ¹H-NMR (CDCl₃, 360MHz): 3.80(ddd, J=4+7+11, 1H); 3.5(m, 2H); 2.48(*d*, J=3, 1H); 2.42(*d*, J=10, 1H); 1.1-1.7(*m*, 8H); 0.96(*d*, J=6, 3H); 0.95(*s*, 3H); 0.93(*t*, J=7, 3H); 0.9-1.0(*m*, 2H); 0.82(*d*, J=6, 3H); 0.73(*s*, 3H).
   - ¹³C-NMR (CDCl₃, 90.6MHz): 92.9(*d*) ; 78.3(*t*) ; 70.6(*d*) ; 40.9(*d*) ; 40.1(*s*) ; 35.2(*t*) ; 34.7(*d*) ; 33.8(*t*) ; 30.2(*t*) ; 26.1(*q*); 19.7(*q*); 18.8(*t*); 15.6(*q*); 14.1(*q*) ; 13.5(*q*).
**(-)-(1'S,2S,3'R,6'R)-1-(2',2',3',6'-Tetramethyl-1'-cyclohexyloxy)-2-pentanol** (IIb)
   - [α_{D}²⁰] = -22.3°: (c=0.046; CHCl₃)
   spectral data identical to (IIIa)

### Example 3

### Stereoselective preparation of the mixture of (1'R,2S,3'S,6'S)-1-(2',2',3',6'-tetramethyl-1'-cyclohexyloxy)-2-pentanol and (1'S,2S,3'R,6'R)-1-(2',2',3',6'-tetramethyl-1'-cyclohexyloxy)-2-pentanol by the Corey reduction method

### 1. (±)-2,2,c-3,t-6-Tetramethyl-R-1-cyclohexanol

The reaction was carried out in a 1.5 l double-walled Schmizo flask fitted with a mechanical stirrer, an adding funnel, a condenser and a thermometer in an argon atmosphere, in which 500 ml of toluene and 22.5 g of metallic sodium (1.0 mol) were heated to reflux for a period of 15 min. The molten two-phase toluene/metal mixture was cooled during vigorous stirring (mechanical stirring with Medimex^{®} transmission) and the metal solidified in the form of small spheres. At 0°, a solution of 54 g of 2,2,3,6-tetramethyl-cyclohexanone (0.35 mol) in 150 g of isopropanol (2.5 mol) was added dropwise over a period of 3 h. The reaction mixture was stirred at 0° overnight, and gas-liquid chromatography (Supelcowax^{®} column, 100°-220°, 15°/min.) showed conversion of 95%. The remaining sodium was destroyed by the slow addition of ethyl alcohol, then the mixture was added to 1000 ml of water. The phases were separated and the aqueous phase extracted twice with 100 ml of toluene. The combined organic phases were dried over sodium sulphate and evaporated to produce 54 g of concentrate. The equatorial/axial ratio according to gas-liquid chromatography (SP-2100, 100°-220°, 15°/min.) was 85:15 and the proportion of the two isomers was 89% of the raw volatile products. Bulb-to-bulb distillation of a sample revealed the absence of non-volatile materials, the yield of raw product therefore being approximately 87%. The product was used such as it was for the following steps.

### 2. (±)-R-2-Allyloxy-1,1,t-3,c-6-tetramethylcyclohexane

A one-litre receptacle in an argon atmosphere was filled with 30 g of a dispersion of KH at 20% in mineral oil (approximately 0.15 mol). The dispersion was diluted with 50 ml of a fraction of petroleum ether at 50°-70°, stirred, decanted, then the liquid drawn off with a pipette. Repetition of this operation ensured the removal of the majority of the mineral oil. 100 ml of THF were then added, and 20 g of 2,2,3,6-tetramethyl-1-cyclohexanol (0.13 mol), obtained under **1**., in 10 ml of THF were added dropwise over a period of 30 min. during stirring (mechanical stirring with Medimex^{®} transmission). Stirring at ambient temperature was continued for 1 h before the mixture was cooled to 0°. A solution of 23 g of allyl bromide (0.19 mol) in 100 ml of dimethylsulphoxide (DMSO) was added over a period of 1 h while maintaining the reaction temperature at 0°. The mixture was left for a further hour at 0" after this addition, then poured over 500 ml of iced water. The phases were separated using 3 x 100 ml of pentane. The combined organic phases were washed with 300 ml of aqueous ammonia at 5%, then twice with 200 ml of water, dried over sodium sulphate and evaporated to produce 33 g of raw oil. Distillation in a Vigreux column (15 cm, < 13.3 Pa) produced 23.6 g of colourless liquid with a boiling point of 43°-44° at 13.3 Pa. The purity achieved according to gas-liquid chromatography (SP-2100, 100°-220°, 15°/min.) was 85%. Yield 79%.
Analytical data :
- IR: 3080 (*w*, *broad*); 2921(*s*); 1648(*w*) ; 1456(*s*); 1387(*m*); 1098(*s*).
- MS: 196 (17%, M⁺) ; 138(24%); 123(26%); 111(31%); 109(15%); 97(23%); 96(32%); 95(26%); 83(41%); 81(22%); 69(62%); 41(100%).
- ¹H-NMR: (CDCl₃, 360MHz) 5.96(*dddd*, *J*=5.5+10.5+16+17, 1H); 5.28(*d* with fine structure, *J*=17, 1H) ; 5.12(*d* with fine structure, *J*=10.5, 1H); 4.1(*m*, 2H); 2.42(*d*, *J*=10, 1H); 1.6(m, 2H) ; 1.35-1.15(*m*, 4H) ; 0.97(*s*, 3H) ; 0.95(*d*, *J*=7, 3H) ; 0.82(*d*, *J*=6, 3H) ; 0.76(*s*, 3H).
- ¹³C-NMR: (CDCl₃, 90.6MHz) 135.5(*s*) ; 115.8(*t*); 93.4(*d*); 75.5(*t*) ; 41.0(*d*); 40.2(*s*); 34.7(*d*) ; 33.8(*t*) ; 30.3(*t*); 26.1(*q*); 19.7(*q*) ; 15.6(*q*); 13.5(*q*).

### 3. (±)-2-[(2,2-c-3-t-6-Tetramethyl-R-1-cyclohexyloxy)methyl]oxirane

A 11 flask was filled with 200 ml of dichloromethane and 50 g of m-chloroperbenzoic acid pure to 70% (0.2 mol). The mixture was cooled to 0° and a solution of 19.5 g of allyl ether at 85% (0.1 mol), obtained under **2**., in 200 ml of dichloromethane was added over a period of 1.5 h at 0°. The reaction mixture was left for 1 h to reach ambient temperature, then allowed to stand overnight. Next, the mixture was poured over 600 ml of an aqueous solution of NaOH at 20% and stirred for 0.5 h. The separated organic phase was washed with 100 ml of NaOH aq. at 20%, then with 200 ml of brine, dried (sodium sulphate) and concentrated to produce 25 g of raw oil. After distillation in a 20 cm Widmer column, 19.0 g of a colourless liquid were collected, with a purity of 95% according to the result of gas-liquid chromatography (SP-2100, 100°-220°, 15°/min.); yield 85%.
Analytical data :
- IR: 3055(*w*); 2920(*s*); 1455(s); 1338(*m*) ; 1100(*s*).
- MS: 212 (34%, M⁺); 138(70%); 127(57%); 123(48%); 109(33%); 96(50%); 83(43%); 69(46%); 57(100%).
- ¹H-NMR: (CDCl₃, 360MHz) 3.77(*dd*, *J*=3.5+21, 1H minor diastereoisomer); 3.74(*dd*, *J*=4+21, 1H major diastereoisomer)-; 3.6(*m*, 1H); 3.2(*m*,1H); 2.80(*t*, *J*=5, 1H); 2.60(*dt*, *J*=2.5+5, 1H); 2.42(*d*, *J*=10, 1H); 1.6(*m*, 2H); 1.4-1.1(*m*, 4H); 1.0(*m*, 6H); 0.82(*d*, broad, *J*=5.5, 3H); 0.76(s, 3H major diastereoisomer) ; *0.75(s,* 3H minor diastereoisomer).
- ¹³C-NMR: (CDCl₃, 90.6MHz) 94.0 *&* 93.9(*d*); 75.5 *&* 75.2(*t*); 51.0 *&* 50.9(*d*) ; 44.8 *&* 44.6(*t*); 40.9 *&* 40.8(*d*) ; 40.3(*s*) ; 34.6(*d*) ; 33.7(*t*) ; 30.2(*t*) ; 26.1 *&* 26.0(*q*); 19.6 & 19.5(*q*) ; 15.6(*q*) ; 13.4(*q*).

### 4. (±)-1-(2,2,c-3,t-6-Tetramethyl-R-1-cyclohexyloxy)-2-pentanol

A 500 ml flask in an argon atmosphere was filled with 60 ml of THF, 6 g of epoxide obtained under **3**. (26 mmol) and 0.30 g of copper (I) iodide (1.5 mmol). The mechanically stirred mixture was cooled to 0° and 110 ml of a solution of 0.36 M of ethyl magnesium bromide in THF (40 mmol) were added dropwise over a period of 1.5 h. The resulting mixture was stirred for a further 30 min., then left to heat up to ambient temperature. It was then poured over 400 ml of NH₄Cl aq. sat., decanted with 3 x 100 ml of pentane and washed with 100 ml of brine. After drying over sodium sulphate, the solvents were evaporated to leave 9.3 g of raw oil. Bulb-to-bulb distillation (10³ Pa, furnace temperature 135°) produced 5.7 g of a product pure to 81% (gas-liquid chromatography SP-2100, 100°-220°, 15°/min.), yield 72%. The product was used such as it was for the following step.

### 5. (±)-1-(2,2,c-3,t-6-Tetramethyl-R-1-cyclohexyloxy)-2-pentanone

A sample of 4 g of alcohol obtained under **4**. (13 mmol) pure to 81% dissolved in 20 ml of dichloromethane was added dropwise to a suspension of 15 g of celite and 14 g of pyridine chlorochromate (65 mmol) in 300 ml of dichloromethane at ambient temperature. The mixture was stirred for 30 min. until conversion was complete, and filtered over 200 g of SiO₂. 2.8 g of a product pure to 98% with a yield of 89% was obtained.
Analytical data:
- IR: 2964(s); 1720(s); 1458(m); 1372(m); 1108(s).
- MS: 240 (4%, M⁺) ; 155(22%); 139(39%); 97(14%); 83(100%).
- ¹H-NMR: (CDCl₃, 360MHz) 4.20(*d*, *J*=16, 1H); 4.08(*d*, *J*=16, 1H); 2.54(*t*, *J*=7, 2H); 2.42(*d*, *J*=10, 1H); 1.6(*m*, 4H) ; 1.3(*m*, 2H) ; 1.2(*m*, 2H); 0.95(s, 3H); 0.94(t, *J*=6, 3H); 0.92(*d*, *J*=7, 3H); 0.83(*d*, *J*= 6, 3H) ; 0.80(*s*, 3H).
- ¹³C-NMR: (CDCl₃, 90.6MHz) 209.6(*s*); 94.4(*d*); 80.2(*t*); 41.2(t) ; 40.1(*d*); 40.2(*s*); 34.6(*d*); 33.7(*t*); 30.1(*t*); 26.1(*q*); 19.6(q); 16.7(*t*); 15.6(*q*); 13.8(*q*) ; 13.5(*q*).

### 6. (1'R,2S,3'S,6'S)-1-(2',2',3',6'-Tetramethyl-1'-cyclohexyloxy)-2-pentanol and (1'S,2S,3'R,6'R)-1-(2',2',3',6'-tetramethyl-1'-cyclohexyloxy)-2-pentanol

### a) Preparation of the catalyst

A 150 ml three-necked flask fitted with a Dean-Stark trap and a condenser in an argon atmosphere was filled with 70 ml of toluene and 5.15g of S-α,α-diphenylprolinol (0.020 mol). 1.70 g of trimethylboroxine (0.014 mol) were introduced over a period of 3 min. A white precipitate appeared and 35 ml of toluene were added while the mixture was stirred continuously for 30 min. The mixture was heated to reflux (98°) and the water separated in the trap. Toluene was added several times and the separation of the water continued until it no longer distilled and all the precipitate had disappeared. The yellow solution was cooled and transferred in an argon atmosphere to a calibrated flask. The final volume was 50 ml with a titer of 0.4 M, assuming total selective conversion of the diphenylprolinol.

### b) Reduction

A 200 ml reactor fitted with a cooling jacket and a mechanical stirrer was placed in an argon atmosphere. 25 ml of anhydrous THF were introduced and cooled to 0° (circulation of a cryostatic bath fixed at -1°). 0.7 ml (0.3 mmol) of the 0.4 M catalyst solution obtained under *a)* was added, then 2.4 ml (4.8 mmol) of a 2 M solution of borane.Me₂S in THF. Using a syringe, 1.7 g of 1-(2,2,3,6-tetramethylcyclohexyloxy)-2-pentanone (7.1 mmol) were added over a period of 6 h while maintaining the temperature at 0°. The reaction mixture was then poured over 300 ml of a NaOH aq. solution at 10% and decanted. The aqueous phase was extracted with 2 x 100 ml of pentane. The combined organic phases were washed in brine, dried over sodium sulphate and concentrated under reduced pressure. The residue was distilled (bulb-to-bulb distillation, 125°, 133 Pa) to produce 1.6 g of colourless liquid. Yield 94%.
- [α_{D}²⁰] = -2.6: (c=0.045; CHCl₃)

### Example 4

### Preparation of a perfuming composition

A perfuming composition for a fabric softener was prepared from the following ingredients :

| Ingredients | Parts by weight |
|---|---|
| Benzyl acetate | 10 |
| Citronellyl acetate | 30 |
| Cinnamic alcohol | 25 |
| Fenchyl alcohol at 10% * | 15 |
| Benzoic aldehyde at 10% * | 130 |
| Lenic aldehyde C 11 at 10% * | 30 |
| Aldehyde C12 at 50% * | 20 |
| Hexylcinnamic aldehyde | 405 |
| 2-Methylundecanal at 10% * | 45 |
| Ambrox^{®1)} at 10% * | 20 |
| Methyl anthranilate | 40 |
| γ-Undecalactone | 35 |
| Raspberry ketone at 1% * | 80 |
| Methyl benzoate at 10% * | 45 |
| Camphor | 20 |
| Citronellol | 130 |
| Allylphenoxyacetate | 10 |
| Coumarin | 80 |
| Alpha damascone at 1% * | 40 |
| Ethyl vanillin | 45 |
| Eugenol F | 165 |
| Galaxolide ^{®2)} | 220 |
| Hedione ^{®3)} | 300 |
| Heliotropine | 30 |
| Synth. hydroxycitronellal at 10% * | 80 |
| Iralia ^{®4)} | 490 |
| Lilial ^{® 5)} | 110 |
| Linalol | 20 |
| Lyral ^{®6)} | 75 |
| Mandarin essential oil | 50 |
| Methyl eugenol at 10% * | 50 |
| Cryst. methyl naphthyl ketone | 40 |
| Crystal moss | 40 |
| Oxide of rose | 20 |
| Paracresol extra at 10% * | 20 |
| Patchouli essential oil | 200 |
| Phenethylol ord. | 50 |
| Phenythexanol | 75 |
| Orange essential oil | 20 |
| Amyl salicylate | 75 |
| Benzyl salicylate | 240 |
| Phenyethyl salicylate at 10% * | 50 |
| 3-(Z)-Hexenol salicylate | 20 |
| Hexyl salicylate | 90 |
| Tonalide ^{®7)} | 180 |
| Vanillin at 10% * | 70 |
| Zestover ⁸⁾ | 30 |
| Galbex ^{®9)} at 10% * | 40 |
| α-Terpineol | 95 |
| Total | 4200 |

| | |
|---|---|
| * in dipropylene glycol (DIPG) 1) 8,12-epoxy-13,14,15,16-tetranorlabdanum; origin: Firmenich SA, Geneva, Switzerland 2) 1,3,4,6,7,8-hexahydro-4,6,6,7,8,8-hexamethylcyclopenta-γ-2-benzopyrane ; origin: International Flavors & Fragrances, USA 3) methyl dihydrojasmonate ; origin : Firmenich SA, Geneva, Switzerland 4) mixture of isomers of methyl ionones ; origin : Firmenich SA, Geneva, Switzerland 5) 3-(4-tert-butylphenyl)-2-methylpropanal ; origin: Givaudan-Roure SA, Vernier, Switzerland 6) 4-(4-hydroxy-4-methylpentyl)-3-cyclohexene-1-carbaldehyde + 3-(4-hydroxy-4-methylpentyl)-3-cyclohexene-1-carbaldehyde ; origin: International Flavors & Fragrances, USA 7) (5,6,7,8-tetrahydro-3,5,5,6,8,8-hexamethyl-2-naphthyl)-1-ethanone; origin: PFW, Holland 8) 2,4-dimethyl-3-cyclohexene-1-carbaldehyde; origin: Firmenich SA, Geneva, Switzerland 9) origin : Firmenich SA, Geneva, Switzerland | |

The addition of 100 parts by weight of (+)-(1'R,2S.3'S,6'S)-1-(2',2',6'-tetramethyl-1'-cyclohexyloxy)-2-pentanol intensified the patchouli note of the fragrance of this perfuming composition, imparting to it a more amber-scented, balsamic, almost juicy connotation. When (+)-(1'R,2S,3'S,6'S)-1-(2',2',3',6'-tetramethyl-1'-cyclohexyloxy)-2-pentanol was replaced by (+)-(1'R,2R,3'S,6'S)-1-(2',2',3',6'-tetramethyl-1'-cyclohexyloxy)-2-pentanol, the fragrance of the composition became more amber-scented, cedar-scented, drier and richer.

### Example 5

### Preparation of a perfuming composition for an eau de toilette for men

A base composition for an eau de toilette for men was prepared from the following ingredients :

| Ingredients | Parts by weight |
|---|---|
| Benzyl acetate | 25 |
| Geranyl acetate | 5 |
| Linalyl acetate | 60 |
| Styrallyl acetate | 30 |
| Hexylcinnamic aldehyde | 340 |
| Allyl amyl glycolate | 15 |
| Wormwood essential oil at 10% * | 50 |
| Raspberry ketone at 1% * | 20 |
| Bergamot abergapt essential oil | 100 |
| Cardamom | 5 |
| Cashmeran ¹⁾ | 10 |
| Sfuma lemon essential oil | 100 |
| Citronellol | 110 |
| Coumarin | 20 |
| Cumin oil at 10% * | 50 |
| Cypress essential oil | 90 |
| Alpha damascone at 1% * | 120 |
| Dihydromyrcenol ²⁾ | 290 |
| 1-Allyl-4-methoxybenzene ³⁾ | 25 |
| Eugenol | 10 |
| Phenylethyl formiate at 10% | 20 |
| Synth. juniper essential oil | 100 |
| Geraniol | 15 |
| Hedione^{®} HC ⁴⁾ | 270 |
| Synth. hydroxycitronellal | 60 |
| Indole at 1% * | 40 |
| Iso E super⁵⁾ | 500 |
| Labdanum res. Spain at 10% * | 40 |
| Lavandin grosso | 50 |
| Linalol | 85 |
| Lyral^{® 6)} | 100 |
| α-iso-Methylionone | 170 |
| Crystal moss | 80 |
| Ketone musk | 50 |
| 2-Methyl nonynoate at 10% * | 20 |
| Patchouli essential oil | 400 |
| Phenethylol ord. | 60 |
| Phenylethyl phenylacetate at 10% * | 85 |
| Benzyl salicylate | 110 |
| Sandalore^{®7)} | 20 |
| 5-Methyl-3-heptanone oxime⁸⁾ at 10% * | 50 |
| Tonalide ^{® 9)} | 100 |
| Ylang extra | 20 |
| Total | 3920 |

| | |
|---|---|
| * in dipropylene glycol 1) 1,2,3,5,6,7-hexahydro-1,1,2,3,3-pentamethyl-4-indenone; origin : International Flavors and Fragrances, USA 2) origin : International Flavors and Fragrances, USA 3) origin : Givaudan-Roure SA, Vernier, Switzerland 4) methyl dihydrojasmonate with a high *cis* isomer content ; origin : Firmenich SA, Geneva, Switzerland 5) 1-(octahydro-2,3,8,8-tetramethyl-2-naphthalenyl)-1-ethanone; origin : International Flavors and Fragrances, USA 6) 4-(4-hydroxy-4-methylpentyl)-3-cyclohexene-1-carbaldehyde + 3-(4-hydroxy-4-methylpentyl)-3-cyclohexene-1-carbaldehyde ; origin : International Flavors *&* Fragrances, USA 7) 3-methyl-5-(2,2,3-trimethyl-3-cyclopenten-1-yl); origin: Givaudan-Roure SA, Vernier, Switzerland 8) origin : Givaudan-Roure SA, Vernier, Switzerland 9) (5,6,7,8-tetrahydro-3,5,5,6,8,8-hexamethyl-2-naphthyl)-1-ethanone; origin : PFW, Holland | |

When 300 parts by weight of (+)-(1'R,2S,3'S,6'S)-1-(2',2',3',6'-tetramethyl-1'-cyclohexyloxy)-2-pentanol were added to this base composition, a new composition was obtained, the fragrance of which had a very intense top note of the woody, amber-scented type, accompanied by an almost balsamic, incense sub-note.

The addition of the same quantity of (+)-(1'R,2R,3'S,6'S)-1-(2',2',3',6'-tetramethyl-1'-cyclohexyloxy)-2-pentanol has a different effect on the fragrance of the base composition. The new composition has a woody odoriferous note less intense than the former, but this note is drier, almost piquant and, no longer balsamic.

### Example 6

### Preparation of a perfuming composition for an eau de toilette for women

A base composition for an eau de toilette of an oriental type for women was prepared from the following ingredients :

| Ingredients | Parts by weight |
|---|---|
| Benzyl acetate | 150 |
| Citronellyl acetate | 70 |
| Geranyl acetate | 145 |
| Linalyl acetate | 100 |
| Amylcinnamic aldehyde | 10 |
| Lenic aldehyde C 11 at 10% * | 40 |
| Hexylcinnamic aldehyde | 50 |
| 2-Methyl-1-undecanal at 1% * | 20 |
| Lily-of-the-valley aldehyde¹⁾ at 50% * | 20 |
| Animalis synarome ²⁾ | 10 |
| Synth. bergamot essential oil | 120 |
| Sfuma lemon essential oil | 250 |
| Citronellol | 65 |
| 2-Methyl-4-phenyl-2-butanol | 135 |
| Coumarin | 110 |
| γ-Decalactone at 10% * | 40 |
| Ethyl vanillin | 15 |
| Eugenol | 185 |
| Geraniol | 135 |
| Hedione^{®3)} | 20 |
| Synth. hydroxycitronellal | 290 |
| Isobutyl quinoline ⁴⁾ at 10% * | 10 |
| Isoeugenol extra | 70 |
| Lavender | 90 |
| Linalol | 150 |
| Lyral^{®5)} | 25 |
| Mandarin essential oil | 80 |
| Dalma oakmoss abs.⁶⁾ at 10% * | 80 |
| Musk ketone | 120 |
| Myrrh essential oil | 100 |
| Mandarin aldehyde at 10% ** | 20 |
| Patchouli essential oil | 310 |
| Phenethylol | 260 |
| Phenylethyl phenylacetate at 10% | 10 |
| Phenylethyl pivalate | 105 |
| Orange essential oil | 130 |
| Benzyl salicylate | 475 |
| 3-(Z)-Hexenol salicylate | 110 |
| Styrax essential oil ⁷⁾ | 30 |
| Vanillin | 55 |
| Vertofix heart ⁸⁾ | 300 |
| Mandarinal ⁹⁾ at 10% * | 30 |
| Total | 4630 |

| | |
|---|---|
| * in dipropylene glycol ** in ethyl citrate 1) (3,7-dimethyl-6-octenyloxy)-acetaldehyde ; origin : International Flavors & Fragrances, USA 2) origin : Firmenich SA, Geneva, Switzerland 3) methyl dihydrojasmonate; origin : Firmenich SA, Geneva, Switzerland 4) origin : International Flavors & Fragrances, USA 5) 4-(4-hydroxy-4-methylpentyl)-3-cyclohexene-1-carbaldehyde + 3-(4-hydroxy-4-methylpentyl)-3-cyclohexene-1-carbaldehyde; origin : International Flavors & Fragrances, USA 6) origin : Firmenich SA, Geneva, Switzerland 7) origin : International Flavors & Fragrances, USA 8) origin : International Flavors & Fragrances, USA 9) origin: Firmenich SA, Geneva, Switzerland | |

The addition of 70 parts by weight of a mixture of (1'R,2S,3'S,6'S)-1-(2',2',3',6'-tetramethyl-1'-cyclohexyloxy)-2-pentanol and (1'S,2S,3'R,6'R)-1-(2',2',3',6'-tetramethyl-1'-cyclohexyloxy)-2-pentanol at 1% in dipropylene glycol intensifies the oriental/woody note of the perfume. The patchouli, vanilla and moss notes are much more in evidence, and the perfume gains intensity and diffusiveness.

## Claims

1. As a compound, (1'R,2R,6'S)-1-(2',2',6'-trimethyl-1'-cyclohexyloxy)-2-pentanol.

2. As a compound, one of the isomers of formula

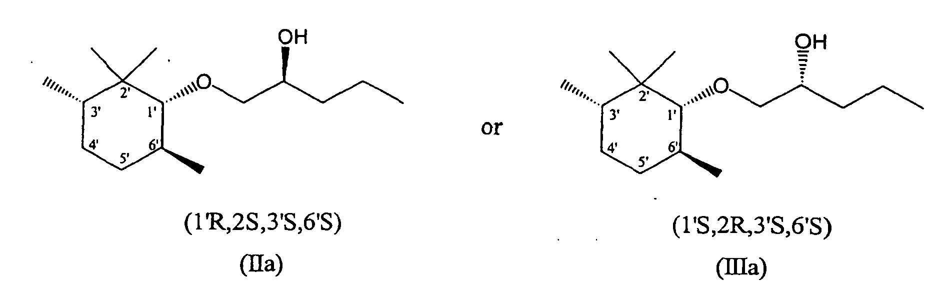

3. A mixture of diastereomers of the formulae

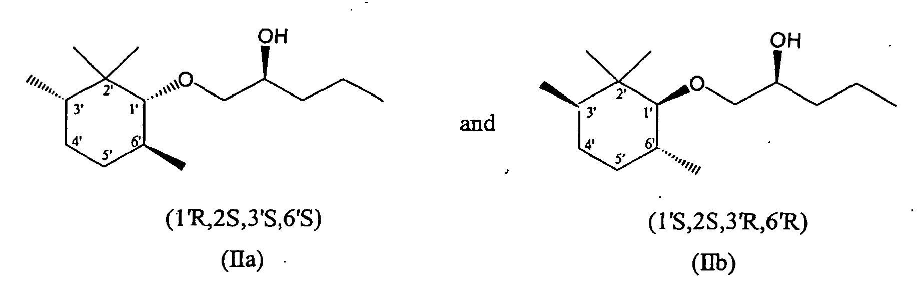

4. Use of a compound according to any one of claims 1 to 3 as an active perfuming ingredient.

5. A perfuming composition or a perfumed article containing a compound according to any one of claims 1 to 3 as active ingredient.

6. A perfumed article according to claim 5, in the form of a perfume or an eau de toilette, a soap, a shower or bath gel, a shampoo or other hair-care product, a cosmetic preparation, a deodorant or air-freshener, a detergent or fabric softener or a cleaning product.

## Patentansprüche

1. Als Verbindung (1'R,2R,6'S)-1-(2',2',6'-Trimethyl-1'-cyclohexyloxy)-2-pentanol.

2. Als Verbindung eines der Isomeren der Formel

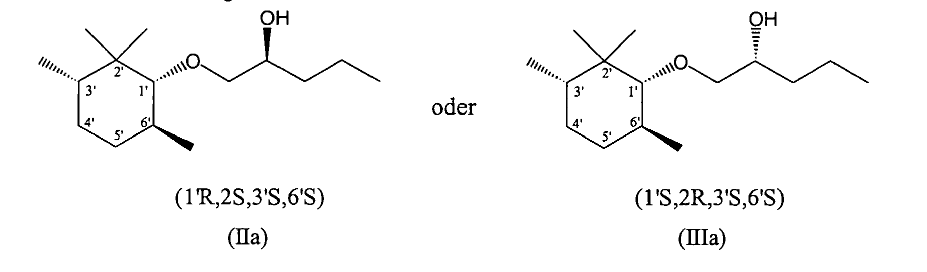

3. Gemisch von Diastereomeren der Formeln

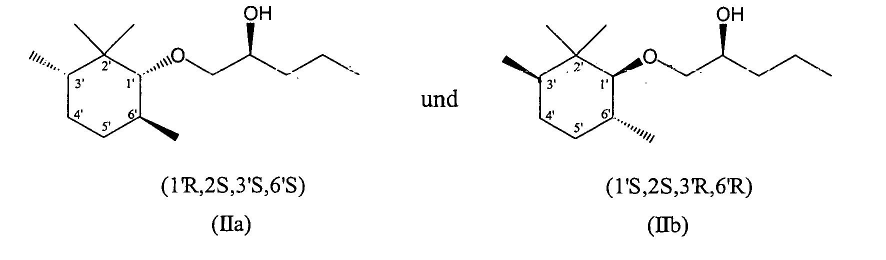

4. Verwendung einer Verbindung nach einem der Ansprüche 1 bis 3 als parfümierender Wirkstoff.

5. Parfümierende Zusammensetzung oder parfümierter Gegenstand, enthaltend eine Verbindung nach einem der Ansprüche 1 bis 3 als Wirkstoff.

6. Parfümierter Gegenstand nach Anspruch 5 in der Form eines Parfüms oder eines Eau de Toilette, einer Seife, eines Dusch- oder Badegels, eines Shampoo oder anderen Haarpflegeprodukts, einer kosmetischen Zubereitung, eines Deodorants oder Luftauffrischers, eines Detergens oder Weichspülmittels oder eines Reinigungsproduktes.

## Revendications

1. En tant que composé, le (1'R,2R,6'S)-1-(2',2',6'-triméthyl-1'-cyclohexyloxy)-2-pentanol.

2. En tant que composé, l'un des isomères de formules

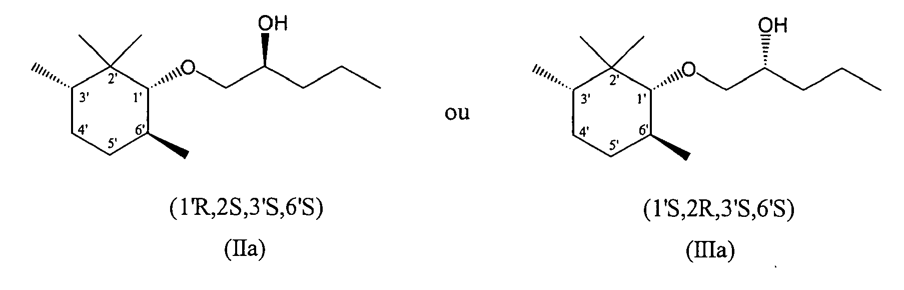

3. Mélange de diastéréomères de formules

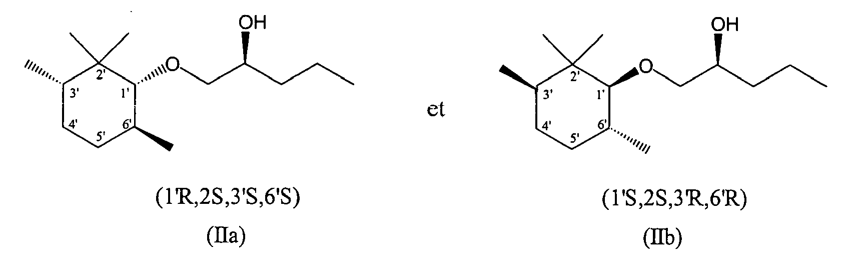

4. Utilisation d'un composé selon l'une quelconque des revendications 1 à 3 à titre d'ingrédient parfumant actif.

5. Composition parfumante ou article parfumé contenant un composé selon l'une quelconque des revendications 1 à 3 à titre d'ingrédient parfumant actif.

6. Article parfumé selon la revendication 5, sous la forme d'un parfum ou d'une eau de toilette, d'un savon, d'un gel de douche ou de bain, d'un shampoing ou d'autre produits de soins capillaires, d'une préparation cosmétique, d'un déodorant ou désodorisant d'air ambiant, ou d'un détergent, assouplissant textile ou produit de nettoyage.
